# DEMANDE DE BREVET EUROPEEN

(11) **EP 1 439 697 A1**
(43) Date de publication de la demande: **21.07.2004**
(21) Numéro de dépôt: 03291099.4
(22) Date de dépôt: 07.05.2003
(51) Int. Cl.: H04N 7/16, H04N 7/167

(54) **Système de reception de données numériques diffusées comprenant un terminal numérique maître, et au moins un terminal numérique esclave**

(30) Priorité: 20.01.2003 FR 0300941
(71) Demandeur: Thomson Licensing S.A., 92100 Boulogne-Billancourt (FR)
(72) Inventeur: Leyendecker, Philippe, 35410 Chateaugiron (FR); Cueff, Jean-Maurice, 92260 Fontenay aux Roses (FR); Creusot, Daniel, 78960 Voisins le Bretonneux (FR)
(74) Mandataire: Berthier, Karine

(57) **Abrégé**

Le système de réception de données numériques diffusées (notamment des services de télévision payants) comprend un terminal numérique maître (1), et au moins un terminal numérique esclave (2) raccordé au terminal maître par une liaison (3) et susceptible de recevoir des données numériques protégées. Le terminal numérique esclave ne peut accéder aux données protégées que si des informations nécessaires pour accéder aux données et reçues par le terminal numérique maître sont transmises par l'intermédiaire de la liaison (3) au terminal numérique esclave dans un délai prédéterminé. Ces informations sont notamment des droits d'accès à des services de télévision ou des clés de désembrouillage des services.

## Description

La présente invention concerne un système de réception de données numériques diffusées comprenant un terminal numérique maître, et au moins un terminal numérique esclave raccordé au terminal maître.

Le marché des décodeurs de télévision numériques arrive actuellement à un tournant. La plupart des abonnés, dans les pays Européens notamment, sont équipés d'un seul terminal numérique (ou « décodeur ») par foyer alors qu'ils possèdent souvent au moins deux téléviseurs. Il existe donc une demande pour des équipements multiples en termes de décodeurs pour un même foyer.

On notera que dans la suite les termes « décodeur » ou « terminal numérique » désignent un même type de dispositif permettant de recevoir et de décoder (et éventuellement désembrouiller) des signaux numériques diffusés par un opérateur (notamment un opérateur de télévision numérique). On utilisera aussi dans la suite de la description les termes « embrouiller » / « désembrouiller » ou « chiffrer » / « déchiffrer » pour signifier que l'on applique un algorithme de cryptage / décryptage à des données en utilisant une clé.

Certains opérateurs de télévision numérique payante souhaitent offrir à leurs abonnés la possibilité de s'équiper de plusieurs terminaux numériques pour bénéficier de leurs services sur chacun des téléviseurs installés dans leur logement, sans pour autant leur faire payer pour les terminaux supplémentaires le prix d'un abonnement plein tarif, qui serait prohibitif, mais plutôt un tarif réduit (voire nul). Cependant il s'agit, pour l'opérateur, de s'assurer que les terminaux et abonnements « associés » restent effectivement dans le même foyer, car dans le cas contraire, ses revenus risquent d'en être considérablement affectés.

Une solution connue consiste à utiliser la « voie de retour » des terminaux numériques en demandant à l'abonné de relier tous les terminaux de son domicile à une même ligne téléphonique. L'opérateur contrôle ensuite périodiquement la connexion des terminaux à cette ligne téléphonique en télécommandant des appels téléphoniques des terminaux vers un serveur de l'opérateur. Cependant cette solution n'est pas satisfaisante car elle impose la connexion permanente des terminaux numériques de l'abonné à une ligne téléphonique.

Une autre solution décrite dans la demande de brevet français No. 02 09362 déposée le 24 juillet 2002 par le même demandeur que la présente demande, THOMSON Licensing S.A., consiste à garantir qu'un lien de communication physique existe toujours entre un terminal secondaire (ou terminal « esclave ») et un terminal principal (ou terminal « maître ») avec lequel il est appairé. Le ou les terminaux esclaves (pour lequel ou lesquels l'abonné bénéficie d'un tarif préférentiel) ne peuvent fonctionner, c'est à dire fournir des données en clair au téléviseur auquel ils sont raccordés, sans qu'il soit vérifié que le terminal « maître » auquel ils sont appairés est présent à proximité.

Plusieurs stratégies de communication entre ces décodeurs sont envisageables mais certaines peuvent présenter des risques de « piratage » ou de « contournement ».

Un but de la présente invention est d'apporter un perfectionnement à l'invention décrite dans la demande de brevet précitée en minimisant les risques de piratage ou de contournement

Le principe de l'invention est le suivant : un terminal numérique « maître » contient une carte à puce dans laquelle sont enregistrés des droits payés par l'abonné au tarif normal. Un terminal numérique « esclave » contient une carte à puce dont les droits, identiques ou non à ceux de la carte à puce du décodeur « maître », ont été payés moins cher par le même abonné.

Ce tarif préférentiel de l'abonnement du décodeur « esclave » est accordé par l'opérateur à la condition que le décodeur esclave soit utilisé par le même abonné dans le même logement que le décodeur « maître ».

L'idée de base à l'origine de l'invention consiste à considérer que si le terminal numérique « esclave » n'est pas à proximité immédiate du terminal numérique « maître », il est utilisé dans un logement différent et donc l'abonné viole le contrat lui permettant de bénéficier d'un tarif préférentiel. Grâce à la présente invention, si une telle situation d'utilisation frauduleuse du terminal numérique « esclave » est détectée, ce dernier cesse de fonctionner normalement ; en l'occurrence, il ne permet plus à l'abonné d'accéder à l'ensemble des services qu'il est censé recevoir (image et son).

On notera que l'invention peut être mise en oeuvre entre un terminal numérique maître et plusieurs esclaves, si l'opérateur le permet.

L'invention concerne à cet effet un système de réception de données numériques diffusées comprenant un terminal numérique maître, et au moins un terminal numérique esclave raccordé au terminal maître par une liaison et susceptible de recevoir des données numériques protégées. Selon l'invention, le terminal numérique esclave ne peut accéder aux données protégées que si des informations nécessaires pour accéder auxdites données et reçues par le terminal numérique maître sont transmises par l'intermédiaire de ladite liaison au terminal numérique esclave dans un délai prédéterminé.

Les données numériques protégées sont notamment des services de télévision embrouillés par des clés et les informations pour accéder aux données protégées sont notamment des messages contenant des droits d'accès aux services ou bien des paramètres permettant d'extraire de tels messages des données reçues ou bien encore des messages contenant une partie des droits d'accès.

Dans un mode de réalisation particulier de l'invention, les informations nécessaires pour accéder aux données protégées qui sont reçues par le terminal numérique maître proviennent du système de diffusion des données.

Avantageusement, les informations pour accéder aux données reçues par le terminal numérique maître sont transformées avant d'être transmises au terminal numérique esclave.

Dans un autre mode de réalisation particulier, les informations nécessaires pour accéder aux données protégées qui sont reçues par le terminal numérique maître proviennent du terminal numérique esclave et sont transformées avant d'être retransmises au terminal numérique esclave.

L'opération de transformation dans les modes de réalisation ci-dessus comprend notamment un désembrouillage et/ou déchiffrement des informations dans le terminal numérique maître, le désembrouillage / déchiffrement étant effectué à l'aide de clés reçues au préalable par le terminal numérique maître du système de diffusion.

Selon une caractéristique particulière de l'invention, le délai prédéterminé est décompté à partir de l'envoi par le terminal numérique esclave d'un message au terminal numérique maître.

Selon une autre caractéristique, le délai prédéterminé est décompté à partir de l'envoi par le système de diffusion des données d'un message au terminal numérique maître.

L'invention concerne aussi un terminal numérique destiné à recevoir des données numériques protégées et qui ne peut accéder aux dites données protégées que si des informations nécessaires pour accéder aux dites données, et reçues par autre terminal numérique auquel il est susceptible d'être raccordé, lui sont transmises par cet autre terminal dans un délai prédéterminé.

Pour résumer, le mécanisme de base de l'invention est le suivant :
- le terminal numérique maître reçoit une partie des éléments nécessaires au désembrouillage des services par le terminal numérique esclave ;
- ces éléments sont transmis au terminal numérique esclave dans des conditions bien définies et de manière unique par l'intermédiaire d'une liaison physique de communication entre les deux terminaux ;
- si le terminal numérique maître n'est pas en mesure de fournir ces éléments au terminal numérique esclave dans un délai prédéterminé, le terminal numérique esclave n'est pas capable d'accéder au service reçu.

L'invention sera mieux comprise à la lecture de la description détaillée qui va suivre de plusieurs modes de réalisation. Cette description est donnée uniquement à titre d'exemple et se réfère aux dessins annexés sur lesquels :
La figure 1 représente un schéma synoptique d'un système selon l'invention.
La figure 2 illustre un premier mode de réalisation de l'invention.
La figure 3 illustre un second mode de réalisation de l'invention.
La figure 4 illustre un troisième mode de réalisation de l'invention.
La figure 5 illustre un quatrième mode de réalisation de l'invention.
La figure 6 illustre une variante du second mode de réalisation.
La figure 7 illustre une variante du quatrième mode de réalisation.

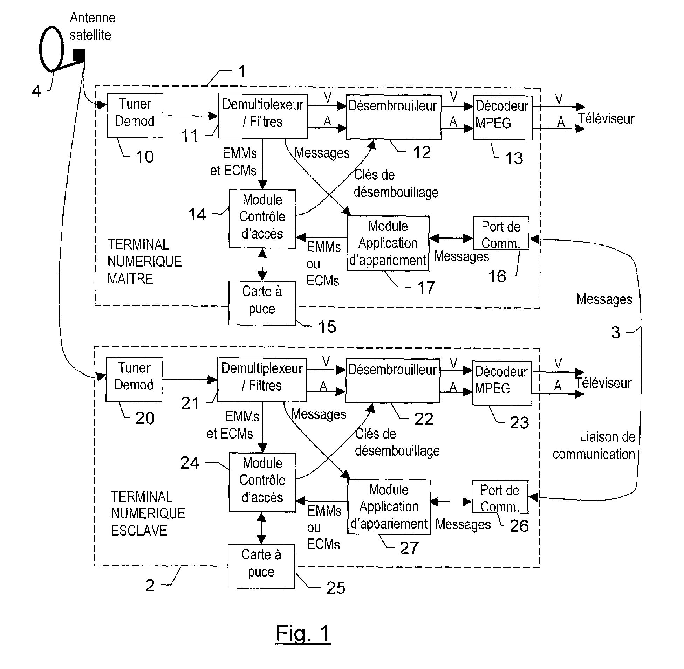

Sur la figure 1, nous avons représenté deux terminaux numériques (ou décodeurs) : un terminal maître 1 et un terminal esclave 2, qui sont reliés par une liaison de communication 3. Les deux terminaux reçoivent, par l'intermédiaire d'une antenne satellite 4, des données numériques diffusées par un opérateur de service, notamment des données audio/vidéo. Ils comportent chacun une carte à puce 15 / 25 insérée dans un lecteur de carte du terminal et dans laquelle sont stockés des droits de l'abonné pour accéder aux services (notamment aux chaînes diffusant des programmes audiovisuels) de l'opérateur.

Les données reçues sont embrouillées, selon le principe classique de la télévision numérique payante, par des clés d'embrouillage (appelées souvent « Control Word ») et les clés sont elles-mêmes chiffrées et transmises dans des messages notés ECM (acronyme de « Entitlement Control Message » signifiant « Message de contrôle des droits ») avec les données liées au service. Des messages personnalisés, notés EMM (de « Entitlement Management Message » signifiant « Message de gestion des droits ») permettent de mettre à jour sur chaque carte à puce les « droits » dont dispose chaque abonné (ces droits pouvant également être reçus par une ligne téléphonique de l'abonné à laquelle est relié le terminal, comme dans le cas du « Pay per View » - ou « payement à la séance » - par exemple).

Pour désembrouiller un service auquel un abonné a droit, les ECMs sont envoyés à un module de contrôle d'accès 14 / 24 qui, en liaison avec la carte à puce 15 / 25, fournit les clés de désembrouillage déchiffrées correspondantes, ces clés permettant de désembrouiller le service. La carte à puce 15 / 25 contient en effet les éléments nécessaires (tels que clés et algorithmes de déchiffrement) pour déchiffrer les clés de désembrouillage contenues dans les messages ECMs. Les clés de désembrouillage sont dynamiques et changent au plus toutes les 10 secondes. Cette période pendant laquelle une clé de désembrouillage spécifique est valide pour désembrouiller les données est appelée « key period » ou « crypto-période ».

On notera que le module de contrôle d'accès 14 / 24 et la carte à puce 15 / 25 ne sont qu'un exemple d'implémentation du système de contrôle d'accès dans les terminaux 1 / 2. Le module 14 / 24 peut être mis en oeuvre dans un module détachable, lui-même contenant éventuellement une carte à puce ou un processeur sécurisé et destiné à être raccordé au décodeur (par exemple un module selon la norme DVB-Cl, de « Digital Video Broadcasting - Common Interface » ou selon la norme NRSS-B, de « National Renewable Security Standard »). De même, la carte à puce 15 / 25 amovible peut être remplacée par un processeur sécurisé intégré dans le terminal 1 / 2.

Sur la figure 1, les données numériques embrouillées sont reçues par un tuner/démodulateur 10 / 20 dans chaque terminal 1 / 2. Un démultiplexeur et dispositif de filtrage 11 / 21 extrait des données reçues les messages ECMs et EMMs qui sont dirigés vers le module de contrôle d'accès 14 / 24. Ce module 14 / 24, en liaison avec la carte 15 / 25, déchiffre les clés de désembrouillage pour les transmettre à un désembrouilleur 12 / 22, lequel reçoit les données audio/vidéo A / V du module de démultiplexage et de filtrage 11 / 21. Grâce aux clés de désembrouillage reçues du module 14 / 24, le désembrouilleur 12 / 22 peut désembrouiller les données A/ V et les transmettre à un décodeur, notamment un décodeur MPEG 13 / 23 qui restitue en sortie des signaux audio / vidéo en clair pour un téléviseur.

Selon l'invention, un module de gestion de l'application d'appariement 17 / 27 est présent dans le terminal maître 1 et dans le terminal esclave 2. Il gère les communications entre les deux terminaux et en particulier le transfert des informations du terminal maître vers le terminal esclave pour permettre au terminal esclave d'accéder aux données reçues. Ce module contrôle également le délai qui s'écoule avant la réception de ces informations de manière à bloquer le fonctionnement du terminal esclave si les informations ne sont pas reçues dans le délai fixé. Un port de communication 16 / 26 disposé dans chaque terminal gère la liaison entre les deux terminaux.

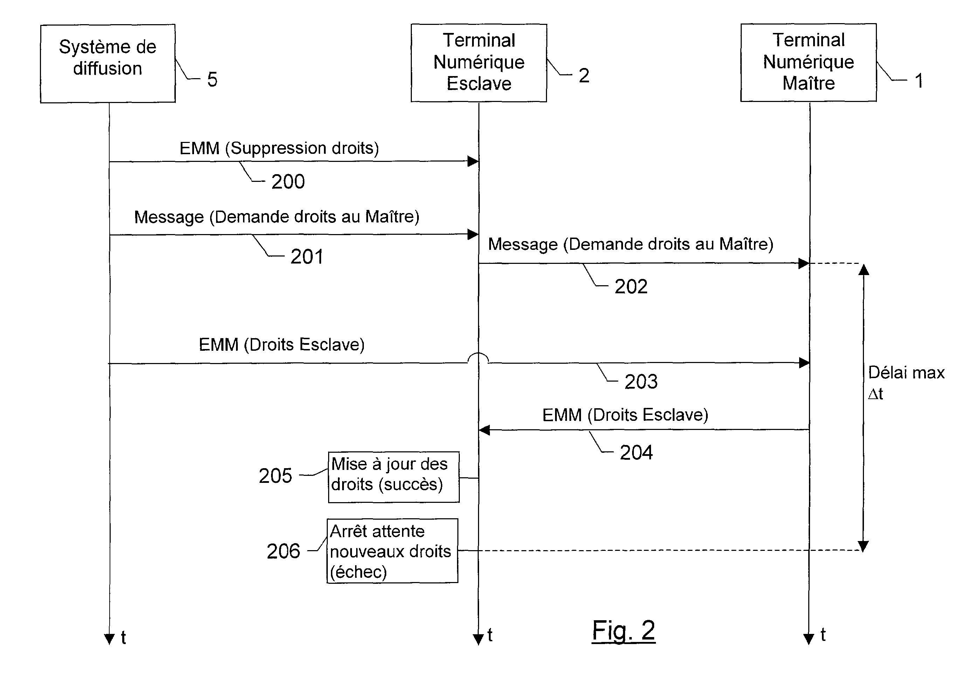

La figure 2 illustre une première méthode d'implémentation de l'invention, basée sur les EMMs.

Elle consiste à fournir les droits (EMMs) du terminal numérique esclave 2 par l'intermédiaire du terminal numérique maître 1 et de la liaison de communication d'appariement 3, et non plus par l'antenne satellite 4. En pratique, lors d'une première étape 200, le terminal numérique esclave 2 reçoit du système de diffusion 5, par satellite, un message *« EMM (Suppression droits) »* qui efface tout ou partie des droits de sa carte à puce 25. Immédiatement après, lors d'une étape 201, il reçoit une information « *Message (Demande droits au Maître) »* qu'il doit transmettre au terminal maître 1 par la liaison physique 3 (étape 202). Le terminal numérique maître utilise cette information pour capter un EMM émis peu de temps plus tard (étape 203). Ce message « *EMM (Droits Esclave)* » est ensuite immédiatement retransmis au terminal numérique esclave par la liaison de communication 3 lors de l'étape 204. Le message « *EMM (Droits Esclave)* » permet au terminal esclave 2 de mettre à jour ses droits dans sa carte à puce à l'étape 205.

Préférentiellement, le message *« EMM (Droits Esclave)* » est transmis lors de l'étape 204 en étant protégé par chiffrement. Par exemple, on suppose que les modules de gestion de l'application d'appariement 17 et 27 présents dans les terminaux 1 et 2 possèdent chacun une clé sécrète partagée par les deux modules 17 et 27. Le module 17 chiffre le message « *EMM (Droits Esclave)* » avec la clé secrète avant de l'envoyer sur la liaison 3 et le module 27 le déchiffre avec la clé secrète lorsqu'il le reçoit. Cette clé secrète partagée peut avoir été reçue du système de diffusion 5 dans des EMMs spécifiques ou peut avoir été programmée dans les terminaux 1 et 2 au moment de leur fabrication ou de leur mise en service.

Selon le principe de l'invention, si la réponse du terminal maître 1 n'est pas reçue dans un délai imparti (délai maximal Δt), le décodeur esclave se bloque (étape 206), jusqu'à la prochaine émission d'EMMs.

On notera que la fréquence d'émission des EMMs peut être faible (un ou plusieurs jours). De plus, le délai maximal imparti Δt doit être suffisamment long pour que les terminaux numériques aient le temps de traiter les informations et suffisamment court pour qu'un retard introduit par un intermédiaire de type réseau Internet soit prohibitif et bloque le terminal esclave. Un délai Δt de l'ordre d'une seconde peut par exemple convenir.

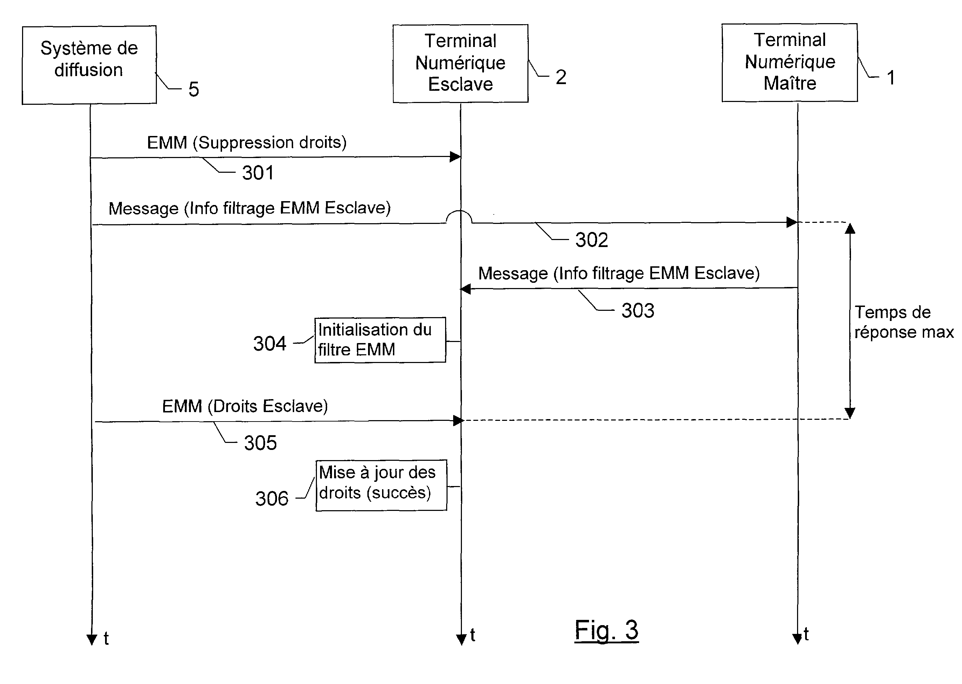

La figure 3 illustre une deuxième méthode d'implémentation de l'invention, également basée sur les EMMs.

Elle consiste à fournir au terminal numérique esclave 2 les informations de filtrage des EMMs par l'intermédiaire du terminal maître 1 et de la liaison de communication d'appariement 3.

Lors d'une première étape 301, le terminal esclave 2 reçoit du système de diffusion 5 un message « *EMM (Suppression droits)* » qui annule tout ou partie des droits de sa carte 25. Immédiatement après, lors d'une étape 302, le terminal maître reçoit et retransmet (étape 303) un message contenant les paramètres de filtrage des EMMs *« Message (Info filtrage EMM Esclave) »* du terminal esclave, ces informations devant être envoyées au terminal esclave par la liaison de communication 3 dans un temps de réponse maximal donné. Le terminal numérique esclave 2 initialise ensuite (étape 304) ses filtres (compris dans le module Démultiplexeur / Filtres 21) avec les paramètres reçus. Préférentiellement, le message transmis à l'étape 303 est protégé par chiffrement de la même manière (exposée ci-dessus) que celle employée pour protéger le message transmis à l'étape 204 de la figure 2.

Lorsque, à l'étape 305, les droits (« *EMM (droits Esclave)* ») sont ensuite diffusés par l'opérateur de services (à partir du système de diffusion 5) vers le terminal esclave 2, celui-ci peut, grâce aux informations reçues du terminal maître, capter l'EMM contenant les droits de la carte « esclave » 25 et mettre à jour ses droits à l'étape 306 pour continuer de fonctionner normalement.

Si le terminal numérique esclave 2 n'a pas reçu les informations de filtrage EMM dans le temps de réponse maximal imparti pour que le terminal maître les retransmette, les droits du terminal esclave 2 ne sont pas restaurés, et il ne fonctionne plus normalement. En pratique, le temps de réponse maximal est décompté au niveau du système de diffusion 5 entre l'émission du *« Message (Info de filtrage EMM Esclave) »* et l'émission du message « *EMM (droits Esclave)* ». Ce temps de réponse maximal est par exemple de l'ordre d'une seconde et peut varier d'un système à l'autre.

D'autres variantes simples peuvent être envisagées : par exemple le terminal maître reçoit du système de diffusion 5 une partie de l'EMM (respectivement de l'ECM) du terminal esclave puis il la retransmet au terminal esclave dans un laps de temps limité.

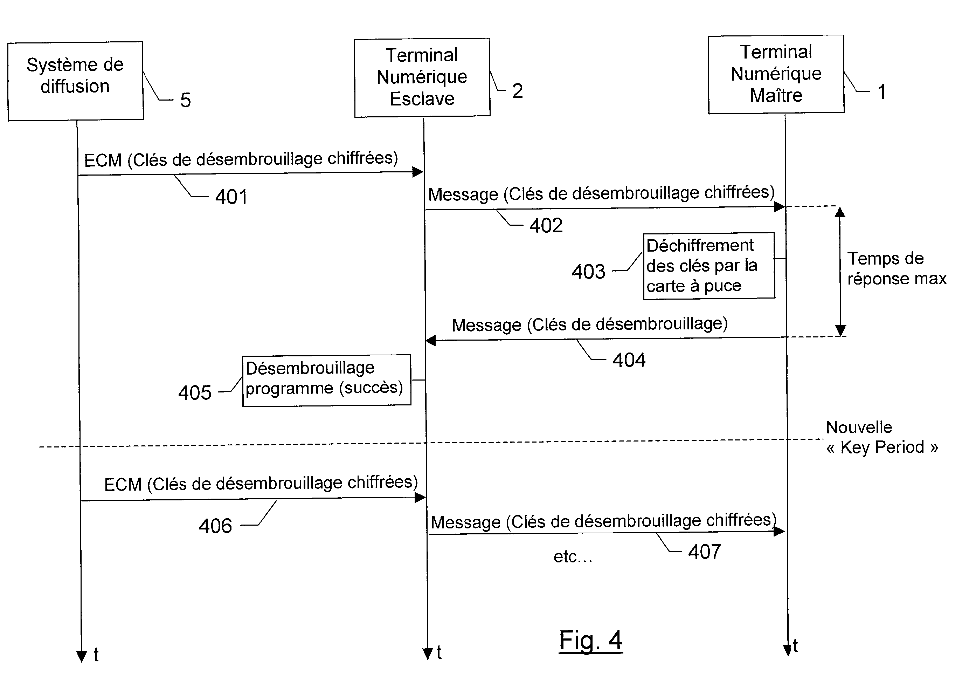

La figure 4 illustre une troisième méthode d'implémentation de l'invention, basée non plus sur les EMMs mais sur les ECMs.

Selon cette méthode, les ECMs contenant les clés de désembrouillage nécessaires pour désembrouiller les données audio/vidéo du programme sélectionné sur le terminal esclave 2 ne sont pas déchiffrés dans le terminal esclave (par le module de contrôle d'accès 24 en liaison avec la carte à puce 25), mais dans le terminal maître 1 (par le module de contrôle d'accès 14 en liaison avec la carte à puce 15). Les éléments (clés et algorithmes) nécessaires pour le déchiffrement des clés de désembrouillage ne sont contenus que dans le terminal maître (plus précisément dans sa carte à puce 15).

En pratique, et comme illustré à la figure 4, lorsqu'un ECM est reçu par le terminal esclave 2 avec le flux de données embrouillées contenant notamment des programmes audiovisuels (étape 401), l'ECM (ou seulement les clés de désembrouillage chiffrées qu'il contient) est immédiatement envoyé au terminal maître 1 par la liaison physique 3 (étape 402). Les clés de désembrouillage sont ensuite déchiffrées à l'étape 403 à l'aide des éléments contenus dans la carte à puce 15. Puis, lors de l'étape 404, les clés de désembrouillage ainsi déchiffrées sont renvoyées au terminal esclave 2 qui peut ainsi initialiser le désembrouilleur 22 pour la prochaine crypto-période (ou « key-period »). Le désembrouillage des programmes peut ainsi avoir lieu avec succès à l'étape 405.

Si en revanche les clés de désembrouillage déchiffrées ne sont pas reçues à temps par le terminal esclave 2, celui-ci ne peut pas désembrouiller les données contenant les programmes qu'il reçoit.

L'opération décrite ci-dessus est répétée pour chaque crypto-période (ou « key period ») et les étapes 406 et 407 correspondent aux étapes 401 et 402 respectivement.

Préférentiellement, le message transmis à l'étape 404 contenant les clés de désembrouillage déchiffrées est protégé par un chiffrement local entre le terminal maître 1 et le terminal esclave 2 de la même manière (exposée ci-dessus) que celle employée pour protéger le message transmis à l'étape 204 de la figure 2.

Un laps de temps limité (noté « Temps de réponse max » sur la figure 4), qui peut varier d'un système à l'autre et qui est par exemple de l'ordre d'une seconde, peut en outre être imposé entre l'envoi (étape 402) par le terminal esclave 2 des messages contenant les clés de désembrouillage chiffrées au terminal maître 1 et la réception (étape 404) des clés déchiffrées par le terminal esclave 2. Cette contrainte permet de limiter les possibilités de contournement par Internet.

Les implémentations décrites ci-dessus impliquent certaines contraintes d'utilisation du terminal maître : il doit être actif et en mesure de recevoir les EMMs/ECMs/messages en permanence, d'une part puisque la diffusion des informations par le système de diffusion n'est pas prédictible dans le temps et d'autre part parce que le système de diffusion n'a pas de retour d'information sur le fait que ces EMMs/ECMs/messages ont été reçus par leurs destinataires.

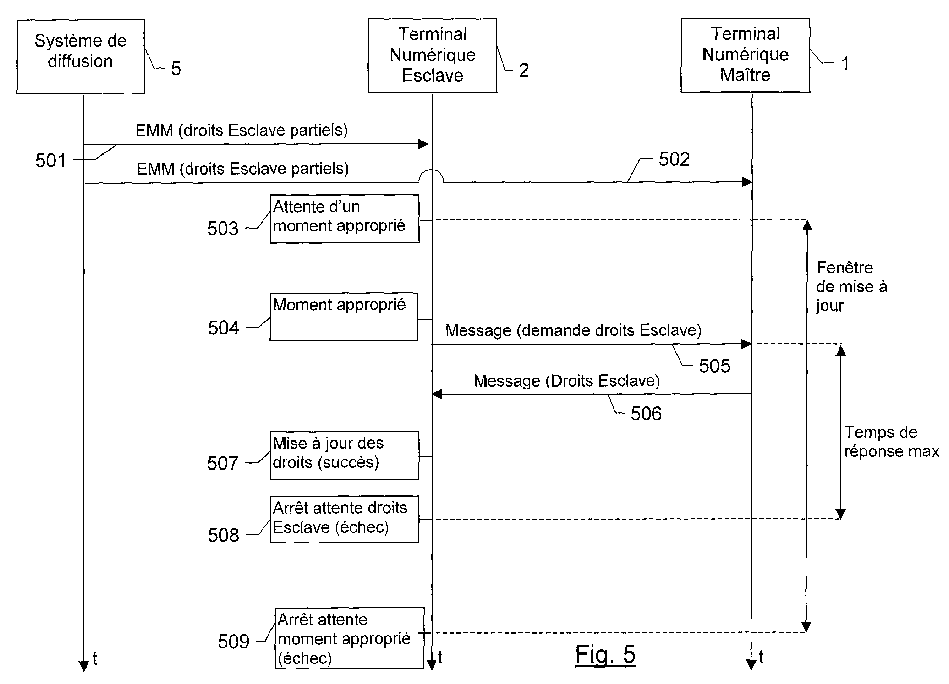

La quatrième méthode d'implémentation de l'invention qui suit, illustrée par la figure 5, permet de réduire ces contraintes.

Selon ce mode de réalisation de l'invention, tout ou partie des informations permettant au terminal esclave 2 de constituer ses droits sont reçues sous forme d'EMM que nous appellerons *« EMM (droits Esclave partiels)* » et mémorisés par le terminal maître 1. Le terminal esclave 2 va demander au terminal maître ces informations à un moment ultérieur.

Sur la figure 5, à l'étape 501 le terminal esclave 2 reçoit du système de diffusion 5 un EMM contenant une partie des informations permettant de reconstituer ses droits et à l'étape 502 le terminal maître 1 reçoit un EMM contenant des informations, complémentaires de celles transmises au terminal esclave 2 à l'étape 501, pour reconstituer les droits du terminal esclave. Naturellement, les étapes 501 et 502 peuvent être effectuées simultanément ou dans un ordre inverse.

Le moment où se fait l'échange d'informations entre les deux terminaux est préférentiellement choisi de manière à garantir que cet échange sera un succès (par exemple juste après avoir vérifié que la communication entre les 2 décodeurs est opérationnelle et/ou s'être assuré de la présence de l'abonné près de son terminal esclave pour qu'il puisse suivre d'éventuelles instructions). L'étape notée 503 sur la figure 5 représente cette attente d'un moment approprié pour le transfert des droits partiels du terminal esclave. L'opération de transfert des droits doit cependant avoir lieu pendant un intervalle de temps limité, correspondant à la « fenêtre de mise à jour » dans la figure 5 (par exemple quelques jours) après l'arrivée des EMMs, sinon le module logiciel 27 du terminal esclave annule les droits de sa carte à puce 25.

Le moment approprié venu (étape 504), le terminal esclave 2 demande l'information EMM au terminal maître 1 en lui envoyant un « *Message (demande droits Esclaves) »* lors de l'étape 505. Le terminal maître 1 doit renvoyer cette information sous la forme d'un *« Message (Droits Esclave) »* (envoyé à l'étape 506 sur la figure 5) dans un délai maximum de quelques dizaines de millisecondes (« temps de réponse max » sur la figure 5). Si les droits partiels complémentaires de l'esclave sont reçus dans ce délai, alors la mise à jour des droits du terminal esclave 2 est effectuée avec succès (étape 507). En revanche, si cette information n'est pas reçue dans le délai « Temps de réponse max », le terminal esclave cesse d'attendre de nouveaux droits (étape 508) et le module de gestion de l'application d'appariement 27 du terminal esclave annule les droits de sa carte à puce 25. Préférentiellement, le message envoyé à l'étape 506 est protégé par un chiffrement comme cela a été vu précédemment pour les autres exemples d'implémentation.

Lorsque la fenêtre de mise à jour expire sans qu'un moment approprié pour le transfert n'ait été détecté, le module logiciel 27 du terminal esclave annule également les droits contenus dans sa carte à puce 25 (étape 509).

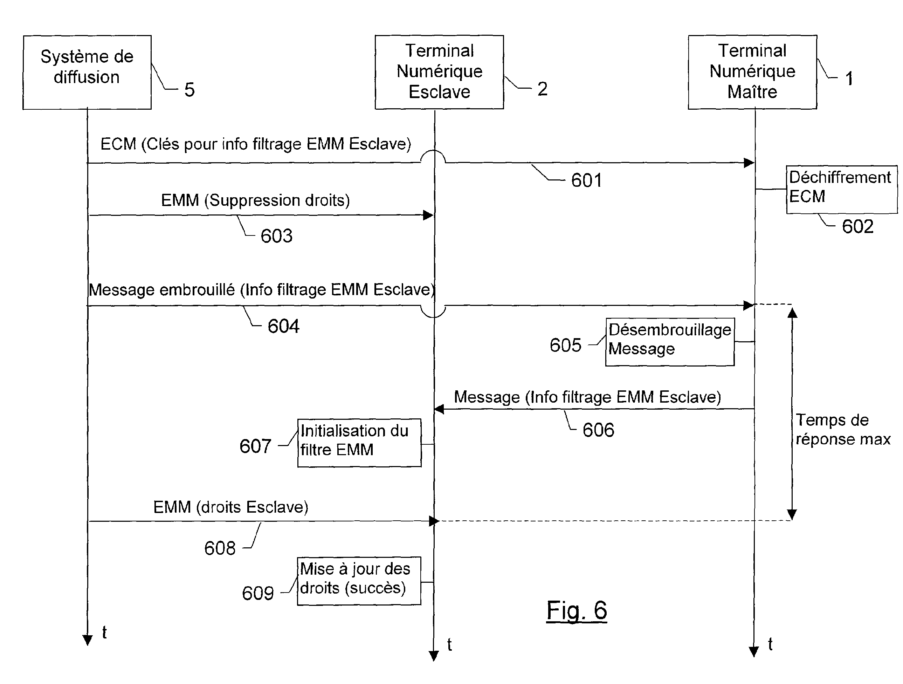

La cinquième méthode d'implémentation suivante de l'invention qui est illustrée par la figure 6 permet de réduire un risque lié à l'émulation possible des messages envoyés par le terminal maître au terminal esclave par un dispositif extérieur.

Les informations qui sont fournies au terminal esclave sont extraites du flux diffusé par le système de diffusion par le terminal maître. Dans les deux premières implémentations illustrées par les figures 2 et 3, l'information reçue par le terminal maître 1 doit être transférée au terminal esclave 2 immédiatement après réception. Un dispositif pirate pourrait être tenté de retrouver une corrélation entre le message circulant sur la liaison de communication 3 et le contenu du flux transport diffusé reçu par le terminal maître dans les instants qui ont précédé, et ainsi être capable de reproduire le processus de traitement du flux transport pour générer un message identique pour le terminal esclave dans un délai suffisamment court. Ce dispositif pourrait être soit un ordinateur équipé d'un tuner / démodulateur / démultiplexeur, soit l'équivalent d'un autre décodeur avec un logiciel adapté, et être mis à proximité du terminal esclave, loin du terminal maître.

Pour éviter qu'une telle corrélation puisse être trouvée, les informations reçues par le terminal numérique maître 1 doivent être transformées, selon cette implémentation préférée de l'invention, avant d'être envoyées au terminal esclave 2. Le moyen le plus sûr disponible dans un terminal numérique pour effectuer cette transformation est l'utilisation du désembrouilleur DVB 12 / 22 sur la figure 1.

Dans la pratique, le système de diffusion envoie au terminal maître 1 un ECM spécial, qui contient une clé de désembrouillage spécifique destinée à désembrouiller un message envoyé ultérieurement au terminal maître 1. Ce message ECM est protégé de manière connue en soi par chiffrement. Lorsque l'ECM est reçu par le terminal maître 1, il est déchiffré dans la carte à puce maître 15, pour obtenir la clé de désembrouillage spécifique. Le message contenant les informations pour le terminal esclave 2 est ensuite envoyé au terminal maître 1 dans des paquets de données embrouillées avec cette clé spécifique. Le terminal maître désembrouille ces paquets de données à l'aide de la clé spécifique reçue précédemment. Une fois désembrouillés, les paquets peuvent être traités par le terminal maître 1 pour générer le message à destination du terminal esclave 2.

Cette méthode est applicable à toutes les variantes d'implémentation citées plus haut. Sur la figure 6, elle est appliquée à la deuxième méthode d'implémentation de l'invention.

Lors de l'étape 601, l'ECM contenant des clés de désembrouillage spécifiques est envoyé par le système de diffusion 5 au terminal maître 1, puis il est déchiffré par le terminal maître à l'étape 602 pour obtenir les clés de désembrouillage. Ensuite, les étapes 603 à 609 sont similaires aux étapes 301 à 306 décrites précédemment en liaison avec la figure 3, à l'exception du fait que le message contenant les informations de filtrage de l'EMM Esclave, envoyé au terminal maître lors de l'étape 604, est envoyé dans des paquets de données embrouillées à l'aide des clés spécifiques reçues précédemment, puis est désembrouillé lors d'une étape supplémentaire 605 dans le terminal maître 1. On notera également que l'étape 603 qui intervient après les étapes 601 et 602 sur la figure 6, peut aussi avoir lieu juste avant l'étape 601 ou entre les étapes 601 et 602.

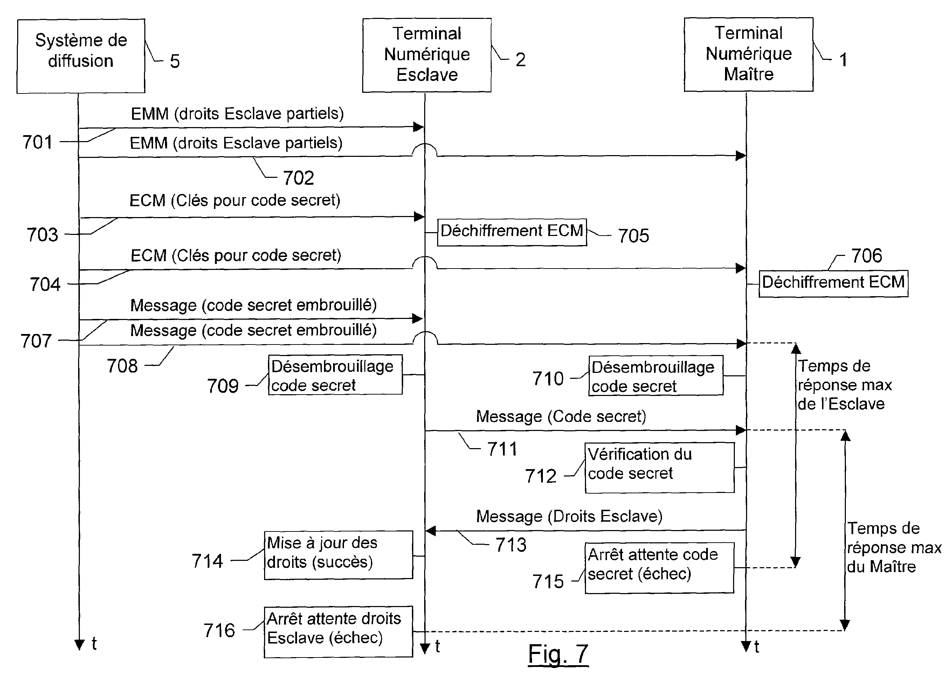

La figure 7 illustre quant à elle une autre variante d'implémentation permettant de faire face à un autre risque. Ce risque identifié en particulier pour le quatrième type d'implémentation (décrit précédemment en liaison avec la figure 5) est celui de l'émulation par un dispositif externe des messages (de type « *Message (demande droits Esclave)* ») envoyés par le terminal esclave 2 au terminal maître 1 pour récupérer les informations partielles stockées dans le terminal maître 1 permettant de reconstituer les droits du terminal esclave.

Un dispositif externe connecté au terminal maître pourrait ainsi émuler la demande du terminal esclave et intercepter la réponse du terminal maître. Cette réponse pourrait ensuite être envoyée par Internet à un autre dispositif externe relié au terminal esclave, qui pourrait alors fournir la bonne information lorsque le terminal esclave la demande.

Pour éviter une telle émulation, on peut proposer soit l'utilisation d'un protocole sécurisé avec authentification, soit plus simplement utiliser, comme dans des variantes précédentes, les ressources de la carte à puce et du système de diffusion.

Selon le principe de cette variante d'implémentation, le système de diffusion 5 envoie à un moment donné (ici, après avoir envoyé les messages EMM contenant les informations permettant de reconstituer les droits du terminal esclave lors d'étapes 701 et 702 - qui correspondent aux étapes 501 et 502 de la figure 5) au terminal maître 1 et au terminal esclave 2 un ECM spécial, contenant une ou des clé(s) de désembrouillage d'un code secret. Cet ECM est envoyé au terminal esclave lors d'une étape 703 et au terminal maître lors d'une étape 704. L'ECM reçu par chaque terminal est ensuite déchiffré dans la carte à puce 15 / 25 de chaque terminal (étapes 705 et 706) pour obtenir la ou les clé(s) de désembrouillage du code secret. Puis le système de diffusion 5 envoie à chacun des terminaux aux étapes 707 et 708 un message identique (« *Message (code secret embrouillé)* »), embrouillé avec ces clés préalablement reçues. Les messages contenant le code secret sont désembrouillés dans chaque terminal 1 / 2 à l'aide des cartes à puces 15 / 25 et du désembrouilleur 12 / 22 lors d'étapes 709 et 710. Le terminal esclave 2 envoie alors au terminal maître 1 un message contenant le code secret obtenu (étape 711).

Le terminal maître 1 attend ce message pendant un laps de temps limité indiqué sur la figure 7 par « Temps de réponse max de l'Esclave ». S'il le reçoit à temps, il vérifie lors d'une étape 712 qu'il s'agit bien du code secret attendu en le comparant à celui qu'il a lui-même reçu, puis, en cas de vérification positive, il répond en envoyant au terminal esclave 2 un message contenant les informations nécessaires pour reconstituer les droits du terminal esclave (étape 713). Le terminal esclave 2 peut alors mettre à jour ses droits sur sa carte à puce 25 avec succès (étape 714). Si le terminal maître 1 n'a pas reçu le message attendu contenant le code secret dans le temps imparti (étape 715) ou bien si le message reçu du terminal esclave 2 ne contient pas le code secret que le terminal maître a reçu au préalable du système de diffusion 5, il n'envoie pas les informations pour reconstituer les droits de l'esclave.

Une fois son message envoyé, le terminal esclave 2 attend lui aussi la réponse du terminal maître 1 pendant un laps de temps limité indiqué sur la figure 7 par « Temps de réponse max du Maître ». Si l'information n'arrive pas dans les délais impartis (étape 716), alors le terminal esclave 2 ne remet pas les droits de sa carte à puce à jour.

Un tel dispositif permet donc, d'une part de rendre non-prédictible l'échange d'informations, et d'autre part impose la contrainte de temps réel qui évite un contournement potentiel par Internet.

On peut aussi, dans une autre variante, utiliser le principe décrit à la figure 7 dans une autre implémentation que celle consistant à envoyer des EMMs contenant des informations partielles pour reconstituer les droits du terminal esclave. On peut notamment prévoir, à intervalles réguliers (par exemple, chaque semaine ou chaque jour), que le système de diffusion 5 envoie des messages ECMs tels ceux envoyés aux étapes 703 et 704 au terminal maître 1 et au terminal esclave 2. Les étapes 707 à 712 se déroulent de la même manière qu'à la figure 7, puis, en cas de vérification positive du code secret à l'étape 712, le terminal maître envoie un message signifiant que le code reçu est correct. Si ce message est reçu après l'expiration du « Temps de réponse max du Maître » ou si le code reçu n'est pas correct, on prévoit dans ce cas que le terminal esclave supprime lui-même les droits contenus dans sa carte à puce 25.

L'invention n'est pas limitée aux méthodes d'implémentation qui ont été décrites ci-dessus. Une autre variante peut notamment être envisagée dans les modes de réalisation illustrés par les figures 2, 3 et 6. Dans toutes ces méthodes d'implémentation, on peut, au lieu d'envoyer un message « *EMM (Suppression droits)* » au début du protocole pour effacer les droits du terminal esclave, attendre la fin du protocole et, si le délai prédéterminé s'est écoulé sans que le terminal esclave n'ait reçu les informations nécessaires du terminal maître, alors on peut prévoir que le terminal esclave supprime lui-même ses droits (par exemple en les effaçant de sa carte à puce 25).

Les avantages de l'invention sont les suivants : comme elle se base sur des éléments de sécurité du système de diffusion lui-même (les informations échangées entre les terminaux sont chiffrées avec des secrets gérés par le système de diffusion des données et par les cartes à puces des terminaux numériques), le risque de piratage au niveau de la carte à puce ou du terminal numérique est réduit.

D'autre part, comme l'invention peut s'appuyer sur l'aspect « temps réel » de l'implémentation, le risque de prolongation de la liaison physique entre deux terminaux numériques par un réseau téléphonique ou Internet est considérablement réduit. En effet, la liaison physique entre les deux terminaux numériques maître et esclave pourrait être « rallongée » indéfiniment par une liaison Internet : l'opérateur de service n'aurait alors plus la garantie que les deux terminaux se trouvent dans le même foyer d'un abonné. En imposant, selon le principe de l'invention, un délai maximum pour le transfert des données, on s'assure ainsi que les informations ne transitent pas par une liaison de type Internet.

Un autre avantage de l'invention est qu'elle garantit que chaque échange de données est différent du précédent, et donc non-prédictible. En effet, un pirate pourrait être tenté d'espionner les informations qui sont reçues par les terminaux pour émuler les informations attendues de la part du terminal numérique maître par le terminal numérique esclave à l'aide d'un dispositif pirate (un ordinateur par exemple). Comme les informations qui sont échangées entre les terminaux changent à chaque communication, elles sont non-prédictibles et ne peuvent donc être facilement émulées par un dispositif pirate.

## Revendications

1. Système de réception de données numériques diffusées comprenant
un terminal numérique maître (1), et
au moins un terminal numérique esclave (2) raccordé au terminal maître par une liaison (3) et susceptible de recevoir des données numériques protégées,
**caractérisé en ce que** ledit terminal numérique esclave ne peut accéder aux dites données protégées que si des informations nécessaires pour accéder aux dites données et reçues par le terminal numérique maître sont transmises par l'intermédiaire de ladite liaison (3) au terminal numérique esclave dans un délai prédéterminé.

2. Système selon la revendication 1, **caractérisé en ce que** les informations nécessaires pour accéder aux données protégées qui sont reçues par le terminal numérique maître (1) proviennent du système de diffusion des données (5).

3. Système selon la revendication 2, **caractérisé en ce que** lesdites informations pour accéder aux données reçues par le terminal numérique maître (1) sont transformées avant d'être transmises au terminal numérique esclave (2).

4. Système selon la revendication 1, **caractérisé en ce que** les informations nécessaires pour accéder aux données protégées qui sont reçues par le terminal numérique maître (1) proviennent du terminal numérique esclave (2) et sont transformées avant d'être retransmises au terminal numérique esclave (2).

5. Système selon l'une des revendications 3 ou 4, dans lequel la transformation comprend un désembrouillage et/ou déchiffrement desdites informations dans le terminal numérique maître (1), le désembrouillage/déchiffrement étant effectué à l'aide de clés reçues au préalable par le terminal numérique maître (1) du système de diffusion.

6. Système selon l'une des revendications précédentes, dans lequel les données numériques protégées comprennent des services de télévision embrouillés par des clés et dans lequel les informations nécessaires pour accéder aux dites données appartiennent à l'ensemble comprenant :
- un message *(EMM (Droits Esclave))* contenant des droits d'accès aux services pour le terminal numérique esclave (2) ;
- un message *(Message (info filtrage EMM Esclave))* contenant des paramètres pour extraire du flux de données reçu par le terminal numérique esclave (2) un message contenant des droits d'accès aux services pour le terminal numérique esclave ;
- un message *(Message (Droits Esclave))* contenant des information partielles permettant au terminal numérique esclave (2) de reconstituer ses droits d'accès aux services ;
- un message *(Message (clés de désembrouillage))* contenant des clés pour le désembrouillage desdites données numériques protégées.

7. Système selon l'une des revendications précédentes, dans lequel le délai prédéterminé est décompté à partir de l'envoi par le terminal numérique esclave (2) d'un message *(Message (Demande droits au Maître) ; Message (Clés de désembrouillage chiffrées); Message (demande droits Esclave); Message (Code secret))* au terminal numérique maître (1).

8. Système selon l'une des revendications 1 à 6, dans lequel le délai prédéterminé est décompté à partir de l'envoi par le système de diffusion (5) des données d'un message *(Message (Info filtrage EMM Esclave) ; Message embrouillé (Info filtrage EMM Esclave))* au terminal numérique maître (1).

9. Système selon l'une des revendications précédentes, dans lequel les informations nécessaires pour accéder aux données protégées sont transmises du terminal numérique maître (1) au terminal numérique esclave (2) en étant protégées par un chiffrement utilisant une clé partagée par les deux terminaux (1, 2).

10. Système selon la revendication 1,
dans lequel le terminal numérique maître (1) et terminal numérique esclave (2) reçoivent en outre du système de diffusion des données (5) un code secret *(Message (code secret embrouillé))* et
dans lequel le terminal numérique maître (1) ne transmet au terminal esclave (2) lesdites informations nécessaires pour accéder aux données que s'il reçoit du terminal esclave (2) ledit code secret *(Message (code secret))* dans un second délai prédéterminé à compter de la réception du code secret par le terminal maître (1).

11. Système selon la revendication 10, dans lequel le code secret reçu par le terminal numérique maître (1) et par le terminal numérique esclave (2) est embrouillé à l'aide de clés transmises au préalable auxdits terminaux par le système de diffusion des données (5).

12. Terminal numérique (2) destiné à recevoir des données numériques protégées, **caractérisé en ce qu'**il ne peut accéder aux dites données protégées que si des informations nécessaires pour accéder aux dites données, et reçues par autre terminal numérique (1) auquel il est susceptible d'être raccordé, lui sont transmises par cet autre terminal dans un délai prédéterminé.
